(19) Europäisches Patentamt
European Patent Office
Office européen des brevets

(11) Veröffentlichungsnummer: **0 000 014**
**A1**

(12) **EUROPÄISCHE PATENTANMELDUNG**

(21) Anmeldenummer: **78100026.0**

(22) Anmeldetag: **01.06.78**

(51) Int. Cl.[2]: **C 07 D 249/12,**
C 07 D 405/04, A 01 N 9/22,
//C 07 C 109/14

(30) Priorität: **03.06.77 DE 2725148**

(43) Veröffentlichungstag der Anmeldung:
**20.12.78 Patentblatt 78/1**

(84) Benannte Vertragsstaaten:
**BE CH DE FR GB NL**

(71) Anmelder: **HOECHST AKTIENGESELLSCHAFT,**
**Postfach 80 03 20,**
**D-6230 Frankfurt/Main 80 (DE)**

(72) Erfinder: **Heubach, Günther, Dr.,**
**Luisenstrasse 15,**
**D-6233 Kelkheim (Taunus) (DE)**

(72) Erfinder: **Emmel, Ludwig, Dr.,**
**An der Oberpforte 1,**
**D-6000 Frankfurt/Main 60 (DE)**

(72) Erfinder: **Waltersdorfer, Anna, Dr.,**
**Geisenheimer Strasse 96,**
**D-6000 Frankfurt/Main (DE)**

(54) **1-Aryl-4-alkyl-1,2,4-triazol-5-onen und Verfahren zu deren Herstellung, sowie deren Verwendung als Schädlingsbekämpfungsmittel.**

(57) Verbindungen der Formel I

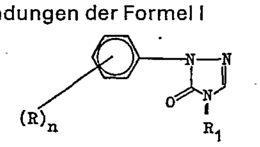

(I)

worin (R)n Wasserstoff, Halogen, Nitro, Cyan (subst.) Alkyl, (subst.) Alkoxy, (subst.) Alkylthio, (subst.) Phenyl, (subst.) Phenoxy oder Methylendioxy und $R_1$ Alkyl, Alkoxyalkyl, Dialkoxyalkyl, Dialkylaminoalkyl oder Cycloalkyl bedeuten, besitzen insektizide, akarizide, fungizide und/oder herbizide Eigenschaften, und werden erhalten durch die Cyclisierung von

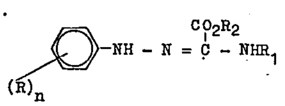

. (II)

mit Phosgen oder Kohlensaüreestern, und Verseifung der erhaltenen

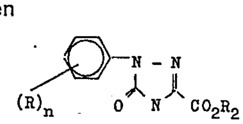

(III)

EP 0 000 014 A1

HOECHST AKTIENGESELLSCHAFT HOE 77/F 114 Dr.Tg/hka

BEZEICHNUNG GEÄNDERT siehe Titelseite

1-Aryl-4-alkyl-1,2,4-triazol-5-one und Verfahren zu ihrer Herstellung sowie ihre Verwendung

Die vorliegende Erfindung betrifft die neuen Verbindungen der Formel I, worin $(R)_n$ Wasserstoff oder 1 bis 4 gleiche oder verschiedene Reste der Gruppe bestehend aus Halogen, Nitro, Cyan, $(C_1-C_6)$-Alkyl, $(C_1-C_6)$-Alkoxy, $(C_1-C_6)$-Alkylthio (wobei die Alkyl-, Alkoxy- und Alkylthiogruppen ihrerseits durch ein oder mehrere Halogenatome substituiert sein können) oder eine Phenyl- oder Phenoxygruppe (die ihrerseits durch Halogen, Alkyl oder Alkoxy substituiert sein können) oder eine am Benzolring ankondensierte Methylendioxygruppe, und $R_1$ geradkettiges oder verzweigtes $(C_1-C_{18})$Alkyl, $(C_1-C_6)$Alkoxyalkyl, Di-$(C_1-C_4)$alkoxyäthyl, Di$(C_1-C_4)$alkylaminoäthyl oder $(C_5-C_6)$-Cycloalkyl bedeuten sowie ein Verfahren zu deren Herstellung. Die Verbindungen sind vor allem insektizid und akarizid wirksam.

Besonders bevorzugt unter den Verbindungen der Formel I sind solche, in denen $(R)_n$ Fluor, Chlor, Dichlor, Brom, Trifluormethyl, Trifluoräthoxy oder Hexafluorpropoxy, $R_1$ geradkettiges oder verzweigtes Alkyl mit 1 - 6 C-Atomen ist.

Man erhält die Verbindungen, indem man Verbindungen der Formel

BAD ORIGINAL

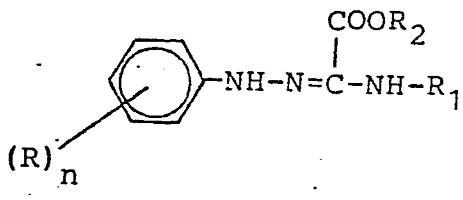

(II)

worin $R_2$ einen Alkylrest bedeutet, mittels Phosgen oder einem niedermolekularen Kohlensäureester cyclisiert, die erhaltenen Verbindungen der Formel

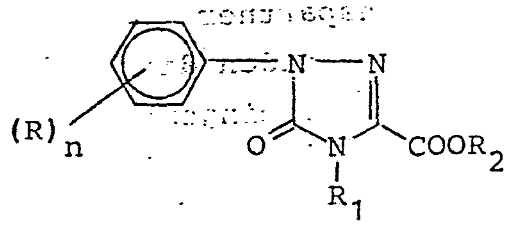

zu den entsprechenden Säuren verseift und letztere decarboxyliert.

Das erfindungsgemässe Verfahren zeichnet sich durch einen überraschend glatten Verlauf aus. Besonders überraschend ist die Tatsache, dass sich die Amidrazone der Formel II glatt und unter sehr milden Bedingungen mit Phosgen bzw. Kohlensäureestern cyclisieren lassen. In Anbetracht der sehr schwach basischen NH-Gruppen in II war ein Ringschluss mit Phosgen bzw. Kohlensäureester nur unter wesentlich energischeren Bedingungen als den brschriebenen zu erwarten. Die besonders leicht und ohne Verwendung von Katalysatoren verlaufende Decarboxylierung der freien Säuren zu den Verbindungen der Formel I war gleichfalls nicht zu erwarten.

Die Umsetzung II ⟶ III erfolgt vorzugsweise bei Temperaturen von -20° bis +6o° C. Die Cyclisierung mit Phosgen wird wie bekannt in Gegenwart von Basen als säurebindenden Mitteln und protonenfreien Lösungsmitteln durchgeführt. Geeignete Basen sind z.B. Triäthylamin, Trimethylamin, N,N-Dimethylanilin oder Pyridin. Als protonenfreie Lösungsmittel kommen besonders Benzol, Äther, Aceton,

Dioxan THF u.ä. in Frage.

Die Reihenfolge der Zugabe der Reaktionspartner ist nicht kritisch. Man kann z.B. alle Reaktionspartner bei tieferer Temperatur vorlegen und dann auf Reaktionstemperatur erwärmen. Man kann auch einen oder zwei Reaktionspartner - z.B. die Verbindung der Formel II und die Base - ganz oder teilweise vorlegen und Phosgen und gegebenenfalls den Rest der Reaktionspartner bei Reaktionstemperatur zugeben. Im allgemeinen werden die Reaktionspartner in etwa stöchiometrischen Mengen eingesetzt bzw. mit einem geringen Überschuss der einen Komponente. Vorzugsweise verwendet man die für die Phosgenierung benötigte Base (vorzugsweise ein tertiäres Amin) in einem stöchiometrischen Überschuss von bis zu 15 % und Phosgen bzw. Kohlensäureester in einem Überschuss bis zu 20 %.

Die gebildete Verbindung der Formel III kann nach Abfiltrieren der Salze durch Abdestillieren des Lösungsmittels isoliert werden. Sie wird mit wässrigem bzw. wässrigalkoholischem Alkali, vorzugsweise NaOH, gegebenenfalls auch ohne Entfernung des Lösungsmittels, verseift, wobei zunächst die Alkalisalze der freien Säure entstehen, die in der wässrigen Phase löslich sind. Die freie Säure wird durch Ansäuern der wässrigen Phase gewonnen und kann durch einfaches Erhitzen, vorzugsweise in einem organischen Lösungsmittel oder auch durch Erhitzen der wässrigen Suspension auf Temperaturen von 80° bis 150° C, vorzugsweise 90° bis 120° C, bzw. beim Siedepunkt des verwendeten Lösungs- bzw. Suspensionsmittels decarboxyliert werden. Das erhaltene Triazolon der Formel I kann durch Entfernen des Lösungsmittels (z.B. Absaugen oder Abdestillieren) isoliert und gegebenenfalls weiter (z.B. durch Umkristallisieren) gereinigt werden.

Die Amidrazone der Formel II erhält man in an sich bekannter Weise, z.B. durch Umsetzung von Phenyldiazoniumsalzen

mit α-Halogenacetessigestern und weitere Umsetzung der erhaltenen α-Halogen-α-phenylhydrazono-glyoxylsäure-ester der Formel

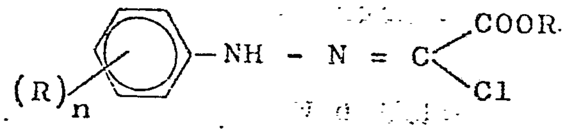 (IV)

mit Aminen der Formel $R_1$-$NH_2$ in an sich bekannter Weise. (Literatur: C. r. 134, 1213 (1902); J. Chem. Soc. 87, 1859 (1905); Ber. 50, 1482 (1917))

Die Verbindungen der Formel I sind wertvolle Schädlingsbekämpfungsmittel. Sie haben vor allem insektizide, insbesondere akarizide und ovizide, daneben zum Teil auch fungizide und herbizide Eigenschaften und sind allein oder in Mischung mit anderen Pflanzenschutzmitteln anwendbar.

Z.B. zeichnen sich die erfindungsgemässen Verbindungen durch sehr gute Wirkung gegen solche Spinnmilbenstämme aus, die gegen chlorierte Kohlenwasserstoffe und Phosphorsäure-derivate resistent geworden sind. Diese Resistenz wird bekanntlich in vielen Ländern und Anbaugebieten zunehmend zu einem ernsten Problem.

Mit den erfindungsgemässen Verbindungen werden sämtliche beweglichen und unbeweglichen Spinnmilbenstadien erfasst.

Besonders gut ist auch die Wirkung gegen Eier, wobei die Rückstandswirkung hervorzuheben ist, die den Neuaufbau von Populationen verhindert. Die ovizide Wirkung erstreckt sich auch auf Eier verschiedener Insektenarten.

Gegenstand der Erfindung sind daher auch Pflanzenschutzmittel, die gekennzeichnet sind durch einen Gehalt an Verbindungen der Formel I.

Die erfindungsgemässen Mittel enthalten die Wirkstoffe gemäss der allgemeinen Formel I zu 2 - 90 %. Sie können als Dispersionen, emulgierbare Konzentrate, benetzbare Pulver, versprühbare Lösungen und Stäubemittel in der üblichen Zubereitungsformen angewendet werden.

Benetzbare Pulver sind in Wasser gleichmässig dispergierbare Präparate, die neben dem Wirkstoff ausser einem Verdünnungs- oder Inertstoff noch Netzmittel, z.B. polyoxäthylierte Alkylphenole, polyoxäthylierte Oleyl- oder Stearylamine, Alkyl- oder Alkylphenyl-sulfonate und Dispergiermittel, z.B. ligninsulfonsaures Natrium, 2,2'-dinaphthylmethan-6,6'-disulfonsaures Natrium oder auch oleylmethyl-taurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten erhalten.

Dispersionen werden durch Suspension des Wirkstoffes in einem organischen Lösungsmittel, wie paraffinische Mineralöle, flüssige Triglyceride und/oder flüssige aromatische Ester, erhalten.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Versprühbare Lösungen, wie sie vielfach in Sprühdosen gehandelt werden, enthalten den Wirkstoff in einem organischen Lösungsmittel gelöst, daneben befindet sich z.B. als Treibmittel ein Gemisch von Fluorchlorkohlenwasserstoffen.

Zur Anwendung werden die handelsüblichen Konzentrate ...

BAD ORIGINAL

0000014

gebenenfalls in üblicher Weise verdünnt, z.B. bei benetzbaren Pulvern, Dispersionen und emulgierbaren Konzentretan mittels Wasser. Staubförmige Zubereitungen sowie versprühbare Lösungen werden vor der Anwendung nicht mehr mit weiteren inerten Stoffen verdünnt. Mit den äusseren Bedingungen wie Temperatur, Feuchtigkeit u.a. variiert die erforderliche Aufwandmenge. Sie kann innerhalb weiter Grenzen schwanken.

HERSTELLUNGSBEISPIELE:

Beispiel 1:

a) α-Chlor-α-(4-chlorphenylhydrazono)-glyoxylsäuremethylester

2,0 Mol (255 g) 4-Chloranilin werden in 652 ml konz. Salzsäure und 1 Liter Eiswasser vorgelegt und in ca. 1 1/2 Stunden zwischen +6° und +8° C mit 140 g Natriumnitrit in 280 ml Wasser versetzt.

40 g Natriumacetat in 1 Liter Eiswasser und 1240 ml Methanol werden mit 2,1 Mol (316 g) α-Chloracetessigsäuremethylester versetzt und zwischen +6° und +8° C in ca. 2 Stunden obige Diazoniumsalzlösung zugetropft. Hierbei wird der pH-Wert durch gleichzeitiges Zutropfen von ca. 470 ml 33 %iger Natronlauge bei 4,5 gehalten (Glaselektrode). Nach Stehen über Nacht war der anfangs ölige Niederschlag kristallin geworden. Die Kristalle werden abgesaugt, mit Wasser gewaschen und in Methanol ausgekocht.

Ausbeute: 444,5 g ≙ 89,9 % d. Th.
Schmp. 149° C

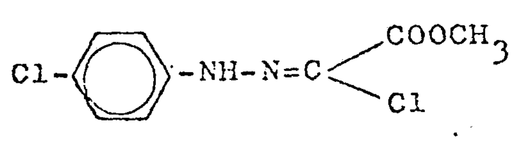

b) <u>α-Isopropylamino-α-(4-chlorphenylhydrazono)-glyoxylsäuremethylester</u>

4,045 Mol (1000 g) α-Chlor-α-(4-chlorphenylhydrazono)-glyoxylsäuremethylester, erhalten nach Beispiel 1 a), werden in 4 Liter Toluol suspendiert und dazu bei +10° C in 2 1/2 Stunden 8,09 Mol (478 g) Isopropylamin unter Rühren getropft. Nachrühren bei Raumtemperatur und Ausrühren mit Wasser ergibt nach Abziehen des Toluols am Rotationsverdampfer bräunliche Kristalle.

<u>Ausbeute:</u> 1080 g ≙ 98,9 % d. Th.
Schmp. 68° - 69° C

Nach Umkristallisieren schmilzt eine Probe bei 70° C. Das Rohprodukt ist für die folgende Umsetzung rein genug.

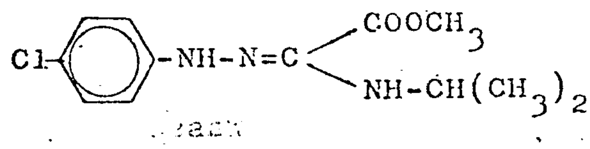

c) <u>1-(4-Chlorphenyl)-3-methoxycarbonyl-4-isopropyl-1,2,4-triazol-5-on</u>

3,42 Mol (921 g) α-Isopropylamino-α-(4-chlorphenylhydrazono)-glyoxylsäuremethylester, erhalten nach Beispiel 1 b), werden in 5 Liter Toluol gelöst und mit 7,9 Mol (798 g) Triäthylamin versetzt. Darauf werden innerhalb von 3 Stunden zwischen -10° und ± 0° C 4,1 Mol (405 g) Phosgen eingegast und 4 Stunden bei Raumtemperatur nachgerührt. Nach Stehenlassen über Nacht rührt man mit Wasser aus und zieht das Lösungsmittel am Rotationsverdampfer ab. Das schmierige Rohprodukt wird aus Methanol (750 ml) umkristallisiert.

<u>Ausbeute:</u> 809 g ≙ 80 % d. Th.
Schmp. 111° - 112,5° C

Eine Analysenprobe wurde nochmals aus Methanol umkristallisiert.

BAD ORIGINAL

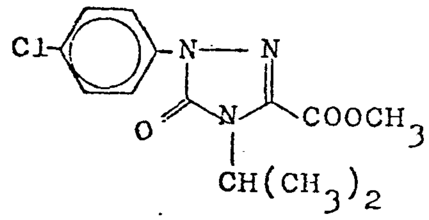

d) 1-(4-Chlorphenyl)-4-isopropyl-1,2,4-triazol-5-on-3-carbonsäure

1,89 Mol (559 g) 1-(4-Chlorphenyl)-3-methoxycarbonyl-4-isopropyl-1,2,4-triazol-5-on, erhalten nach Beispiel 1 c), wurden in 2,7 Liter Methanol suspendiert und auf einmal mit 2,02 Mol (81 g) NaOH in 4 Liter Wasser versetzt. Das Triazol geht in Lösung. Nach einstündigem Kochen am Rückfluss war dünnschicht chromatographisch kein Ester mehr nachweisbar. Nach Abkühlen auf Raumtemperatur und Verdünnen mit Wasser wurde filtriert, das Filtrat mit konz. Salzsäure angesäuert und der Niederschlag mit Wasser gewaschen.

Ausbeute: 516,5 g ≙ 97 % d. Th.
Schmp. 118° C (Zers. $CO_2$)

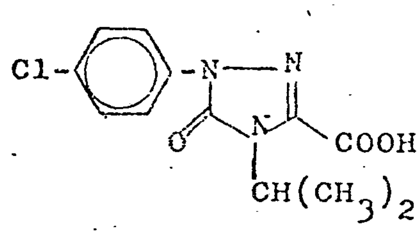

e) 1-(4-Chlorphenyl)-4-isopropyl-1,2,4-triazol-5-on

1,78 Mol (501 g) 1-(4-Chlorphenyl)-4-isopropyl-1,2,4-triazol-5-on-3-carbonsäure, erhalten nach Beispiel 1 d), wurden in 3 Liter Toluol suspendiert und 15 Minuten unter Rückfluss gekocht. Danach war Triazolcarbonsäure dünnschichtchromatographisch nicht mehr nachweisbar. Darauf wurden 2,8 Liter Toluol abdestilliert und der Rückstand mit überschüssigem Benzin versetzt. Nach Absaugen und Waschen mit Benzin erhielt man farblose Kristalle.

Ausbeute: 406 g ≙ 96 % d. Th.
Schmp. 86° C

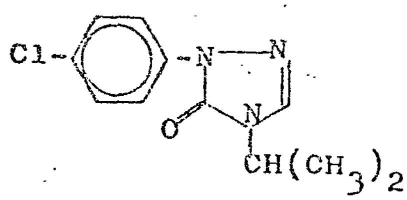

Analog 1 a) bis 1 e) wurden die in der Tabelle I aufgeführten Verbindungen hergestellt:

Tabelle I:

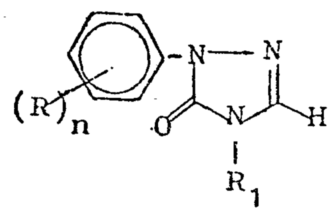

| Beispiel Nr. | (R)n | $R_1$ | Schmp. (° C) |
|---|---|---|---|
| 2 | H | $-CH(CH_3)_2$ | Öl |
| 3 | H | $-CH_2-CH_2-CH_3$ | 43 |
| 4 | H | $-(CH_2)_5-CH_3$ | 73 |
| 5 | H | 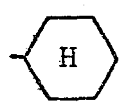 | 107 - 108 |
| 6 | H | $-C(CH_3)_3$ | 103 - 104 |
| 7 | 2-Cl | $-CH(CH_3)_2$ | 118 - 119 |
| 8 | 3-Cl | $-CH(CH_3)_2$ | 80 - 81 |
| 9 | 3-Cl | 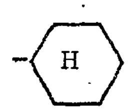 | 102 - 103 |
| 10 | 3-Cl | $-(CH_2)_3CH_3$ | 73 |
| 11 | 3-Cl | $-CH_3$ | 130 - 132 |
| 12 | 3-Cl | $-C(CH_3)_3$ | 102 |
| 13 | 4-Cl | $-CH_3$ | 136 - 137 |
| 14 | 4-Cl | $-(CH_2)_2N(C_2H_5)_2$ | 62 |
| 15 | 4-Cl | 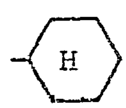 | 153 |

(Fortsetzung)

| Beispiel Nr. | $(R)_n$ | $R_1$ | Schmp. (° C) |
|---|---|---|---|
| 16 | 4-Cl | $-(CH_2)_5CH_3$ | 91 |
| 17 | 4-Cl | $-C_2H_5$ | 96 - 97 |
| 18 | 4-Cl | $-CH_2-CH_2-CH_3$ | 98 - 99 |
| 19 | 4-Cl | $-(CH_2)_{11}CH_3$ | 75 - 77 |
| 20 | 4-Cl | $-CH_2-CH(CH_3)_2$ | 102 - 103 |
| 21 | 4-Cl | $-(CH_2)_3CH_3$ | 93 - 94 |
| 22 | 4-Cl | $-C(CH_3)_3$ | 165 |
| 23 | 4-F | $-CH(CH_3)_2$ | 90 |
| 24 | 4-F | $-CH_2-CH_2-CH_3$ | 57 - 58 |
| 25 | 4-F | 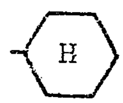 | 118 |
| 26 | 4-Br | $-CH(CH_3)_2$ | 98 - 99 |
| 27 | $3,4-Cl_2$ | $-CH(CH_3)_2$ | 82 |
| 28 | $3,4-Cl_2$ | $-CH_3$ | 152 |
| 29 | $3,4-Cl_2$ | $-C(CH_3)_3$ | 148 |
| 30 | $3,4-Cl_2$ | 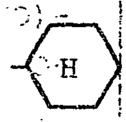 | 134 - 136 |
| 31 | $3,4-Cl_2$ | $-CH_2-CH(CH_3)_2$ | 91 |
| 32 | $3,4-Cl_2$ | $-(CH_2)_3CH_3$ | 78 |
| 33 | $3,4-Cl_2$ | $-CH_2-CH_2-CH_3$ | 110 |
| 34 | $3,4-Cl_2$ | $-CH_2-CH_2OCH_3$ | 122 - 123 |
| 35 | $3,4-Cl_2$ | 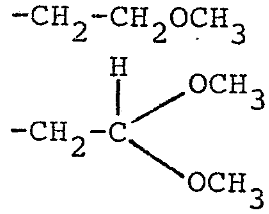 | 99 - 101 |
| 36 | $2,5-Cl_2$ | $-CH(CH_3)_2$ | 104 - 105 |
| 37 | $3,5-Cl_2$ | 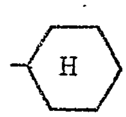 | 165 |
| 38 | $3,5-Cl_2$ | $-CH_2-CH(CH_3)_2$ | 101 - 102 |
| 39 | $3,5-Cl_2$ | $-CH(CH_3)_2$ | 125 - 126 |

BAD ORIGINAL

Tabelle I (Fortsetzung)

| Beispiel Nr. | (R)n | $R_1$ | Schmp. (° C) |
|---|---|---|---|
| 40 | $3-CF_3$ | $-CH(CH_3)_2$ | 88 - 89 |
| 41 | $3-CF_3$ | $-(CH_2)_3CH_3$ | 84 - 85 |
| 42 | $3-CF_3$ | $-CH_3$ | 94 |
| 43 | $3-CF_3$ | $-(CH_2)_2N(C_2H_5)_2$ | 63 |
| 44 | $3-CF_3$ | $-CH_2-CH_2-CH_3$ | 73 - 75 |
| 45 | $3-CF_3$ | 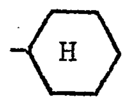 | 121 - 122 |
| 46 | $3-CF_3$ | $-CH_2CH(CH_3)_2$ | 76 - 77 |
| 47 | $3-CF_3$ | $-C(CH_3)_3$ | 110 - 111 |
| 48 | $3-CF_3$ | $-(CH_2)_5CH_3$ | öl |
| 49 | $3-CF_3$ | $-(CH_2)_{11}CH_3$ | 49 - 50 |
| 50 | $3-CF_3$ | $-CH_2-CH_2OCH_3$ | 107 - 108 |
| 51 | $3,5-(CF_3)_2$ | $-CH(CH_3)_2$ | 135 - 136 |
| 52 | $3-CF_3$, 4-Cl | $-CH(CH_3)_2$ | 116 - 117 |
| 53 | $2-CH_3$, 4-Cl | $-CH(CH_3)_2$ | 101 - 102 |
| 54 | $4-CH_3$ | $-(CH_2)_2N(C_2H_5)_2$ | öl |
| 55 | $4-CH_3$ | $-CH(CH_3)_2$ | 81 - 82 |
| 56 | $4-OCH_3$ | $-CH(CH_3)_2$ | 58 - 59 |
| 57 | $3-OCF_3$ | $-CH(CH_3)_2$ | öl |
| 58 | $3-OCF_2CH_2F$ | $-CH(CH_3)_2$ | öl |
| 59 | $3-OCF_2CHFCF_3$ | $-CH(CH_3)_2$ | öl |
| 60 | 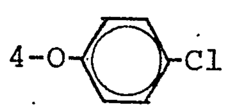 | $-CH(CH_3)_2$ | 105 - 106 |
| 61 | 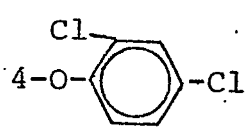 | $-CH(CH_3)_2$ | öl |
| 62 | 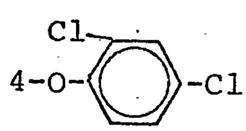 | $-C(CH_3)_3$ | 88 - 89 |
| 63 | $3-S-CH_3$ | $-CH(CH_3)_2$ | 57 - 58 |
| 64 | 2-Cl, $5-CF_3$ | $-CH(CH_3)_2$ | 118 |

Tabelle I (Fortsetzung)

| Beispiel Nr. | $(R)_n$ | $R_1$ | Schmp. (° C) |
|---|---|---|---|
| 65 | 2,4,5-$Cl_3$ | $-CH(CH_3)_2$ | 150 - 151 |
| 66 | 3,4 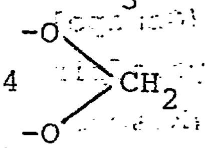 | $-CH(CH_3)_2$ | 111 - 112 |
| 67 | 2,4-$Cl_2$ | $-CH(CH_3)_2$ | 129 - 131 |
| 68 | 3-CN | $-CH(CH_3)_2$ | 133 - 135 |
| 69 | 4-Cl | $-CH_2C_{12}H_{25}$ | Sirup |
| 70 | 4-I | $-CH(CH_3)_2$ | 113 |

Formulierungsbeispiele

Beispiel A:

Ein emulgierbares Konzentrat wird erhalten aus:

15 Gewichtsteilen Wirkstoff
75 Gewichtsteilen Cyclohexanon als Lösungsmittel
10 Gewichtsteilen oxäthyliertes Nonylphenol (10 AeO) als Emulgator

Beispiel B:

Ein in Wasser leicht dispergierbares benetzbares Pulver wird erhalten indem man

25 Gewichtsteile Wirkstoff
64 Gewichtsteile kaolinhaltiges Quarz als Inertstoff
10 Gewichtsteile ligninsulfonsaures Kalium und
1 Gewichtsteil oleylmethyltaurinsaures Natrium als Netz- und Dispergiermittel

mischt und in einer Stiftmühle mahlt.

Beispiel C:

Ein in Wasser leicht dispergierbares Dispersionskonzentrat wird erhalten, indem man

20 Gewichtsteile Wirkstoff mit
6 Gewichtsteilen Alkylphenolpolyglykoläther (Triton X 207®, Rohm und Haas)
3 Gewichtsteilen Isotridecanolpolyglykoläther (Genapol X-080®, Hoechst) und
71 Gewichtsteilen paraffinisches Mineralöl (Essobayol 90®, Esso)

mischt und in einer Reibkugelmühle auf eine Feinheit von unter 5 Mikron vermahlt.

Beispiel D:

Ein Stäubemittel wird erhalten, indem man

10 Gewichtsteile Wirkstoff und
90 Gewichtsteile Talkum als Inertstoff

mischt und in einer Schlagmühle zerkleinert.

## BIOLOGISCHE BEISPIELE

Beispiel I:

Mit der Gemeinen Spinnmilbe (Tetranychus urticae; normal sensibel) stark befallene Bohnenpflanzen (Phaseolus vulgaris) wurden mit der wässrigen Suspension eines Spritzpulverkonzentrates, die 0,05 Gew.-% des Wirkstoffes aus Beispiel 1 enthielt, bis zum Stadium des Abtropfens gespritzt.

Anschliessend erfolgte die Aufstellung der gespritzten Pflanzen im Gewächshaus bei ca. 20° C. Die mikroskopische Kontrolle 8 Tage nach der Spritzung zeigte, dass alle beweglichen und unbeweglichen Stadien der Population, einschliesslich der Eier, getötet waren.

In gleicher Weise geprüft, erwiesen sich auch die Verbindungen gemäss Beispiel 17, 18, 26, 40, 44, 58 und 59 als ebenso gut wirksam.

Beispiel II:

Mit resistenten Obstbaumspinnmilben (Metatetranychus ulmi, Stamm "Dardar") befallene Apfelbäumchen wurden mit der wässrigen Suspension eines Emulsionskonzentrates der in Tabelle II genannten Verbindungen bis zum beginnenden Abtropfen behandelt. Nach der Aufstellung der Pflanzen im Gewächshaus bei ca. 20° C erfolgte nach 8 Tagen die Kontrolle.

Tabelle II:

| Beispiel Nr. | Gew.-% AS in der Spritzbrühe | % Abtötung akarizid | ovozid (geschätzt) |
|---|---|---|---|
| 1 | 0,05 | 100 | 100 |
| 17 | 0,05 | 100 | 100 |
| 18 | 0,05 | 100 | 100 |
| 20 | 0,05 | 100 | 100 |
| 26 | 0,05 | 100 | 100 |
| 46 | 0,05 | 96 | 75 - 100 |
| 48 | 0,05 | 97 | 75 - 100 |
| 58 | 0,05 | 100 | 100 |
| Vergleichsmittel: | | | |
| Azinphosmethyl | 0,1 | 72 | 0 |
| Demeton-S-methyl | 0,1 | 0 | 0 |
| Dimethoat | 0,1 | 75 | 0 |

Beispiel III:

Behandlung von resistenten Spinnmilben (Tetranychus urticae, Stamm "Baardse") wie in Beispiel I angegeben.

Tabelle III:

| Präparat gemäss Beispiel Nr. | Gew.-% AS in der Spritzbrühe | % Abtötung akarizid | ovizid |
|---|---|---|---|
| 1 | 0,1 | 100 | 50 |
| 17 | 0,05 | 100 | 75 - 100 |
| 26 | 0,1 | 94 | 75 |
| 58 | 0,05 | 100 | 50 |
| 59 | 0,05 | 97 | 25 - 50 |
| Vergleichsmittel | | | |
| Demeton-S-methyl | 0,2 | 94 | 0 |
| Dimethoat | 0,2 | 85 | 0 |

0000014

HOE 77/F 114

Patentansprüche:

1. Verbindungen der Formel

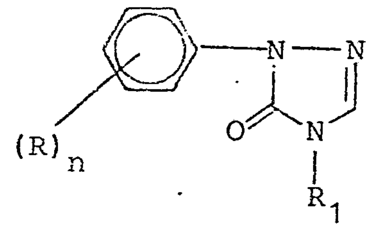

(I)

worin $(R)_n$ Wasserstoff oder 1 bis 4 gleiche oder verschiedene Reste der Gruppe bestehend aus Halogen, Nitro, Cyan, $(C_1-C_6)$-Alkyl, $(C_1-C_6)$-Alkoxy, $(C_1-C_6)$-Alkylthio (wobei die Alkyl-, Alkoxy- und Alkylthiogruppen ihrerseits durch ein oder mehrere Halogenatome substituiert sein können) oder eine Phenyl- oder Phenoxygruppe (die ihrerseits durch Halogen, Alkyl oder Alkoxy substituiert sein können) oder eine am Benzolring ankondensierte Methylendioxygruppe, und $R_1$ geradkettiges oder verzweigtes $(C_1-C_{18})$-Alkyl, $(C_1-C_6)$-Alkoxyalkyl, Di$(C_1-C_4)$alkoxyäthyl, Di$(C_1-C_4)$alkylaminoäthyl oder $(C_5-C_6)$-Cycloalkyl bedeuten.

2. 1-(4-Chlorphenyl)-4-isopropyl-1,2,4-triazol-5-on.

3. 1-(4-Chlorphenyl)-4-äthyl-1,2,4-triazol-5-on.

4. 1-(4-Chlorphenyl)-4-n-propyl-1,2,4-triazol-5-on.

5. Verfahren zur Herstellung von Verbindungen der Formel I, dadurch gekennzeichnet, dass man Verbindungen der Formel

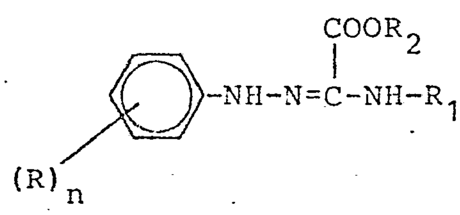

(II)

worin $R_2$ einen Alkylrest bedeutet, mittels Phosgen oder einem niedermolekularen Kohlensäureester cyclisiert, die erhaltenen Verbindungen der Formel

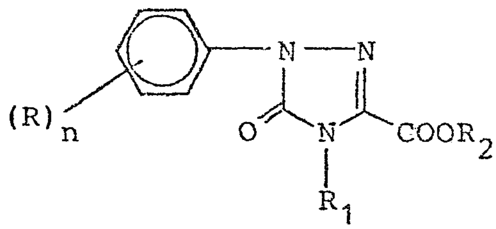

(III)

zu den entsprechenden Säuren verseift und letztere decarboxyliert.

6. Schädlingsbekämpfungsmittel, gekennzeichnet durch einen Gehalt an einer Verbindung gemäss Anspruch 1.

7. Verwendung von Verbindungen gemäss Anspruch 1 zur Bekämpfung von Insekten und Spinnmilben.

Europäisches Patentamt

# EUROPÄISCHER RECHERCHENBERICHT

C000014

Nummer der Anmeldung

EP 78 10 0026

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | Keine Entgegenhaltungen. | |
| | ---- | |

KLASSIFIKATION DER ANMELDUNG (Int.Cl.²)

C 07 D 249/12
C 07 D 405/04
A 01 N 9/22
//C 07 C 109/14

RECHERCHIERTE SACHGEBIETE (Int. Cl.²)

C 07 D 249/12
C 07 D 405/04
A 01 N 9/22
//C 07 C 109/14

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-09-1978 | CREMERS |